# EUROPEAN PATENT APPLICATION

(11) **EP 4 775 677 A1**
(43) Date of publication of application: **15.07.2026**
(21) Application number: 24862951.1
(22) Date of filing: 26.04.2024
(51) Int. Cl.: C12N 15/77, C07K 14/245, C12P 13/10

(54) **MICROORGANISM OF GENUS CORYNEBACTERIUM HAVING ENHANCED L-ARGININE PRODUCTIVITY, AND METHOD FOR PRODUCING L-ARGININE USING SAME**

(30) Priority: 07.09.2023 KR 20230119062
(71) Applicant: Daesang Corporation, Seoul 02586 (KR)
(72) Inventor: RYU, Mi, Seoul 07789 (KR); LEE, Dong Seok, Seoul 07789 (KR); KANG, Dong Hun, Seoul 07789 (KR); BAK, Seong Kun, Seoul 07789 (KR); LEE, Shin Won, Seoul 07789 (KR); YOON, Sun Jun, Seoul 07789 (KR); LEE, Jae Hoon, Seoul 07789 (KR)
(74) Representative: Ipsilon
(86) International application number: PCT/KR2024/005659
(87) International publication number: WO 2025/053367

(57) **Abstract**

The present invention relates to a microorganism of the genus *Corynebacterium* having enhanced L-arginine productivity and a method for producing L-arginine using same. The microorganism of the genus *Corynebacterium* has an improved L-arginine production yield, compared to the parent strain, by having, introduced thereinto, the argO gene encoding an arginine exporter, and thus can effectively produce L-arginine.

## Description

### Technical Field

The present invention relates to a *Corynebacterium* sp. microorganism having improved L-arginine productivity and a method of producing L-arginine using the same.

### Background Art

Arginine is known to be contained in plants or the like in a free state. Although arginine is a non-essential amino acid, it is a semi-essential amino acid that should be necessarily supplied in growing children and special conditions such as stress, trauma, and cancer, and is widely used as a component for amino acid supplements, pharmaceuticals, foods, and the like. For medicine, arginine is used in liver function promoters, brain function promoters, male infertility treatment agents, comprehensive amino acid preparations, and the like, and for food, arginine is used as a fish cake additive, a health drink additive, and a salt substitute for hypertensive patients.

The production of arginine may be performed using a naturally occurring wild-type strain or a mutant strain modified from the wild-type strain so as to have improved arginine productivity. Recently, in order to improve the efficiency of production of arginine, genetic recombination technology has been used for microorganisms such as *Escherichia coli* and *Corynebacterium.* In biosynthesis of L-arginine in microorganisms, L-glutamate is used as a starting material and converted sequentially into N-acetylglutamate, N-acetylglutamyl-P, N-acetylglutamate 5-semialdehyde, N-acetylornithine, L-ornithine, L-citrulline, and argininosuccinate, thereby synthesizing L-arginine. Various proteins such as enzymes, transcription factors, and transport proteins are involved in this stepwise synthesis process. Therefore, there have been attempts to increase the production of L-arginine by inducing mutations in various genes, which are involved in the biosynthetic pathway of L-arginine, or regulatory sequences such as promoters that regulate the expression of these genes, through genetic recombination technology.

According to the literature [Bellmann et al., Microbiology 2001; 147: 1765-74], a lysine exporter is involved in the export of arginine, citrulline, and ornithine as well as lysine, and when the expression of the lysE gene encoding the lysine exporter was enhanced, the ability to export arginine was increased. However, the export of by-products other than arginine was also increased due to the enhancement of the lysE gene, and in particular, the increased production of ornithine, which is a by-product, has a negative effect on arginine production.

Therefore, there is still much research needed to develop recombinant strains or mutant strains that have excellent L-arginine productivity while reducing the production of by-products.

### [Prior Art Documents]

### [Patent Documents]

Korean Patent No. 10-2269637

### DISCLOSURE

### Technical Problem

An object of the present invention is to provide a *Corynebacterium* sp. microorganism having improved L-arginine productivity.

Another object of the present invention is to provide a method of producing L-arginine using the microorganism.

### Technical Solution

One aspect of the present invention provides a *Corynebacterium* sp. microorganism having improved L-arginine productivity by having enhanced activity of an arginine exporter.

The "arginine exporter" used in the present invention serves to regulate intracellular arginine levels and balance arginine and lysine. The arginine exporter in the present invention may be a polypeptide encoded by the argO gene and having arginine exporter activity or the ability to export arginine, without being limited thereto.

Information on the nucleic acid and protein sequences of the arginine exporter is available from known sequence databases (e.g., GenBank, UniProt).

As used herein, the term "enhanced activity" means that expression of genes encoding proteins of interest, such as enzymes, transcription factors and transport proteins, has been newly introduced or has been increased so that the expression levels of the proteins have increased compared to those in a wild-type strain or a strain before modification. The term "enhanced activity" also includes: a case in which the activity of the proteins themselves have increased compared to the activity of the proteins, originally possessed by the microorganism, through substitutions, insertions, deletions or combinations thereof, of one or more nucleotides in nucleotide sequences encoding the genes; a case in which the overall activity of the proteins in the cell is higher than that in the wild-type strain or the strain before modification due to increased expression or translation of the genes encoding the proteins; and a combination thereof.

According to one embodiment of the present invention, enhancement of the activity of the arginine exporter may be achieved by introduction of a gene encoding the arginine exporter.

According to one embodiment of the present invention, the arginine exporter or the gene encoding the same may be derived from an *Escherichia* sp. microorganism.

The *Escherichia* sp. microorganism may be *Escherichia coli, Escherichia albertii, Escherichia blattae, Escherichia fergusonii, Escherichia hermannii,* or *Escherichia vulneris,* without being limited thereto.

For example, the arginine exporter or the gene encoding the same may be derived from *Escherichia coli.*

According to one embodiment of the present invention, the arginine exporter may comprise the amino acid sequence of SEQ ID NO. 1.

According to one embodiment of the present invention, the gene encoding the arginine exporter may comprise the nucleotide sequence of SEQ ID NO. 2.

The amino acid sequence or nucleotide sequence of the arginine ex-transporter according to the present invention may comprise or essentially consist of a sequence having at least 70%, at least 80%, at least 90%, at least 98%, at least 99%, or at least 99.9% homology to the amino acid sequence of SEQ ID NO. 1 or the nucleotide sequence of SEQ ID NO. 2, and should be able to retain its original function.

As used herein, the term "improved productivity" means that L-arginine productivity has increased compared to that of the parent strain. As used herein, the term "parent strain" refers to a wild-type strain or mutant strain to be mutated, and includes a strain that is to be mutated directly or to be transformed with a recombinant vector or the like. In the present invention, the parent strain may be a wild-type *Corynebacterium* sp. microorganism or a *Corynebacterium* sp. microorganism or strain mutated from the wild-type strain.

The Corynebacterium sp. microorganism may be one known in the art, and for example, may be, but is not limited to, *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacterium pseudopelargi,* or *Corynebacterium flavescens.*

According to one embodiment of the present invention, the *Corynebacterium* sp. microorganism may be *Corynebacterium glutamicum.*

The *Corynebacterium* sp. microorganism having improved L-arginine productivity according to the present invention may have an increased ability to export intracellular arginine by having enhanced arginine export activity, and thus may have improved L-arginine productivity. Specifically, the *Corynebacterium* sp. microorganism having improved L-arginine productivity exhibits increased L-arginine productivity compared to the parent strain, and in particular, the amount of L-arginine produced by the *Corynebacterium* sp. microorganism may be at least 1%, 5%, 10%, 15%, 20%, 25%, 30%, 35%, 40%, 45%, 50%, 55%, 60%, 65%, 70%, 75%, 80%, 85%, 90%, 95%, or 100% higher than that produced by the parent strain, or may be 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, or 10-fold higher than that produced by the parent strain, without being limited thereto.

The *Corynebacterium* sp. microorganism according to one embodiment of the present invention may be obtained from the parent strain through a recombinant vector containing a gene encoding the arginine exporter.

As used in the present invention, the term "vector" refers to any type of nucleic acid sequence transfer structure that is used as a means for transferring and expressing a gene of interest in a host cell. Unless otherwise specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. As used herein, the term "operably linked" means that a gene of interest and regulatory sequences thereof are functionally linked together in a manner enabling gene expression, and the "regulatory elements" include a promoter sequence for initiating transcription, any operator sequence for regulating transcription, a sequence encoding suitable mRNA ribosome-binding sites, and a sequence for regulating termination of transcription and translation.

The vector that is used in the present invention is not particularly limited as long as it may replicate in a host cell, and any vector known in the art may be used. Examples of the vector include a natural or recombinant plasmid, cosmid, virus and bacteriophage. Examples of a phage vector or cosmid vector include, but are not limited to, pWE15, M13, AMBL3, λMBL4, λIXII, AASHII, λAPII, λt10, At11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The "recombinant vector" that is used in the present invention may be constructed using a prokaryotic or eukaryotic cell as a host, and may replicate regardless of the genome of the host cell or may be integrated into the genome itself. The host cell may contain a replication origin, which is a particular nucleotide sequence which enables the vector to replicate in the host cell and from which replication starts. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, and T7 promoter), a ribosome binding site for initiation of translation, and a transcription/translation termination sequence. When a eukaryotic cell is used as a host, the vector comprises a replication origin operating in the eukaryotic cell, and examples of the replication origin include, but are not limited to, an f1 replication origin, an SV40 replication origin, a pMB1 replication origin, an adeno replication origin, an AAV replication origin, and a BBV replication origin. In addition, the vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, and HSV-tk promoter), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selection marker include, but are not limited to, ampicillin, kanamycin, streptomycin, and chloramphenicol.

The transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

Where the vector is transformed into prokaryotic cells to generate recombinant microorganisms, examples of host cells that may be used include, but are not limited to, *E. coli* sp. strains such as *E. coli* DH5α, *E. coli* JM109, *E. coli* BL21, *E. coli* RR1, *E. coli* LE392, E. *coli B, E. coli* X 1776, *E. coli* W3110, and *E. coli* XL1-Blue, *Corynebacterium* sp. strains, *Bacillus* sp. strains such as *Bacillus subtilis* and *Bacillus thuringiensis,* and various Enterobacteriaceae strains such as *Salmonella typhimurium, Serratia marcescens,* and *Pseudomonas* species.

Where the vector is transformed into eukaryotic cells to generate recombinant microorganisms, examples of host cells that may be used include, but are not limited to, yeast (e.g., *Saccharomyces cerevisiae*), insect cells, and plant cells and animal cells, such as Sp2/0, CHO K1, CHO DG44, PER.C6, W138, BHK, COS7, 293, HepG2, Huh7, 3T3, RIN, and MDCK cell lines.

As used in the present invention, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed in the host cell, it may be inserted into the chromosome of the host cell, or may exist extrachromosomally, without being limited thereto.

The transformant may include a cell transfected, transformed, or infected with the recombinant vector of the present invention *in vivo* or *in vitro,* and may be used in the same sense as a recombinant host cell, a recombinant cell, or a recombinant microorganism.

Genes inserted into the recombinant vector for transformation according to the present invention may be integrated into a host cell such as a *Corynebacterium* sp. microorganism by homologous recombination crossover.

According to one embodiment of the present invention, the host cell may be a *Corynebacterium* sp. microorganism. The *Corynebacterium* sp. microorganism may be, for example, *Corynebacterium glutamicum.*

Another aspect of the present invention provides a method for producing L-arginine comprising steps of: culturing the *Corynebacterium* sp. microorganism in a medium; and recovering L-arginine from the microorganism or the medium in which the microorganism has been cultured.

The culturing may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For culture media for *Corynebacterium* sp. microorganisms, reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering L-arginine from the cultured *Corynebacterium* sp. microorganism or the medium in which the microorganism has been cultured, the produced L-arginine may be collected or recovered from the medium using a suitable method known in the art depending on the culture method. Examples of a method that may be used to recover the produced arginine include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering L-arginine may be performed by centrifuging the culture medium at a low speed to remove biomass and separating the obtained supernatant through ion-exchange chromatography.

According to one embodiment of the present invention, the step of recovering L-arginine may include a process of purifying the L-arginine.

### Advantageous Effects

The *Corynebacterium* sp. microorganism according to the present invention has an improved L-arginine production yield compared to the parent strain by having introduced thereinto the gene encoding the arginine exporter, and thus is capable of effectively producing L-arginine.

### Mode for Invention

Hereinafter, the present invention will be described in more detail. However, these descriptions are provided for illustrative purposes only to aid in the understanding of the present invention, and the scope of the present invention is not limited by these illustrative descriptions.

### Example 1. Construction of strain into which argO gene has been introduced

To introduce the argO gene, which encodes an arginine exporter derived from an *Escherichia* sp. microorganism, into an arginine-producing strain derived from *Corynebacterium glutamicum, Corynebacterium glutamicum* 14GR (KCCM13219P), which is an L-arginine-producing strain, *E*. *coli* DH5a (HIT Competent cells^{™}, Cat No. RH618) were used.

The *Corynebacterium glutamicum* 14GR was cultured in an ARG-broth medium (pH 7.2) containing 10.5 g of 98% glucose, 1 g of beef extract, 4 g of yeast extract, 2 g of polypeptone, 2 g of NaCl and 40 g of (NH₄)₂SO₄ in 1 L of distilled water at a temperature of 30°C.

The *E. coli* DH5a was cultured on an LB medium containing 10.0 g of tryptone, 10.0 g of NaCl and 5.0 g of yeast extract in 1 L of distilled water at a temperature of 37°C.

The antibiotic kanamycin used was the product of Sigma.

DNA sequencing and gene synthesis were performed by Macrogen, Inc.

### 1-1. Recombinant vector

A recombinant vector was constructed to disrupt the lysE gene, which is involved in the export of lysine and arginine in *Corynebacterium glutamicum* 14GR, and to introduce the argO gene, which encodes an arginine exporter derived from the *Escherichia* sp. microorganisms, into that location. The codon-optimized argO nucleotide sequence (SEQ ID NO: 2) and the right-arm nucleotide sequence used for cloning were obtained through gene synthesis. The left arm portion (655 bp) and the right arm portion (636 bp) with respect to the center of the lysE gene on the *Corynebacterium glutamicum* genome, and the argO gene linked to the promoter were amplified by PCR and ligated together by overlap PCR, followed by cloning into a pk19mobsacB (ATCC, 87098) vector. To construct the plasmid, the primers shown in Table 1 below were used for amplification of each gene fragment.

**[Table 1]**

| Primer name | | Primer sequence (5'→3') | SEQ ID NO. |
|---|---|---|---|
| Amplification primers for left homologous arm of lysE | LF1 | tgattacgcccggatccagatactcctttgga | 3 |
| | LF2 | cggatccagatactcctttgga | 4 |
| | LR1 | ttttggaatcggtggccttc | 5 |
| | LR2 | ttttcgcgggttttggaatc | 6 |
| Amplification primers for promoter | P-F1 | cccgcgaaaatggcgggtattttcatcc | 7 |
| | P-F2 | tggcgggtattttcatccaaa | 8 |
| | P-R1 | tcctccaaaattgtggtggc | 9 |
| | P-R2 | gttcttgtaatcctccaaaattgtgg | 10 |
| Amplification primers for argO and right homologous arm of lysE | F1 | ttacaagaacatgttctcctactacttccaaggc | 11 |
| | F2 | atgttctcctactacttccaaggc | 12 |
| | R1 | attacccttccatcaccatcg | 13 |
| | R2 | gtcaccaacgattacccttcc | 14 |

Using the above-described primers, PCR was performed under the following conditions. PCR was performed for 25 to 30 cycles using a Thermocycler (TP600, TAKARA BIO Inc., Japan) in a reaction solution containing 100 µM of each deoxynucleotide triphosphate (dATP, dCTP, dGTP, dTTP), 1 pM oligonucleotide, 10 ng of the chromosomal DNA of *Corynebacterium glutamicum* ATCC 13032 as a template, and 1 unit of pfu-X DNA polymerase mixture (Solgent). PCR was performed under the following conditions: (i) denaturation at 94°C for 30 sec, (ii) annealing at 58°C for 30 sec, and (iii) extension at 72°C for 1 to 2 min (a polymerization time of 2 min per kb).

The gene fragments prepared as described above were cloned into a pk19mobsacB vector by self-assembly cloning. The vector was transformed into *E. coli* DH5a which was then plated on an LB-agar plate containing 50 µg/ml of kanamycin, and cultured at 37°C for 24 hours. The finally formed colony was isolated and whether the insert was exactly present in the vector was checked. Next, the vector was isolated and used for recombination of the *Corynebacterium glutamicum* strain.

As a process commonly performed in the above method, the corresponding genes were amplified by PCR from the genomic DNA of *Corynebacterium glutamicum* ATCC 13032 and inserted into the pk19mobsacB vector by the self-assembled cloning method according to the strategy, and the resulting plasmid was selected in *E coli* DH5a. For chromosomal base substitution, the gene fragments were amplified individually and ligated together by overlap PCR, thereby preparing the desired DNA fragment. For genetic manipulation, Ex Taq polymerase (Takara) and Pfu polymerase (Solgent) were used as PCR polymerases, and various restriction enzymes and DNA modifying enzymes purchased from NEB were used, and they were used according to manufacturer's provided buffers and protocols.

### 1-2. Corynebacterium glutamicum mutant strain DS1

Mutant strain DS1 was constructed using a cloning vector. The cloning vector was prepared at a final concentration of 1 µg/µl or more and electroporated into *Corynebacterium glutamicum* 14GR (see Tauch et al., FEMS Microbiology letters 123 (1994) 343-347) to induce first recombination. In this case, the electroporated strain was plated on an agar medium containing 50 µg/µl of kanamycin, colonies were isolated, and then whether the vector was properly inserted at the desired position on the genome was checked by PCR and sequencing. Each of the isolated strains was inoculated again in a liquid medium to induce second recombination, cultured overnight or more, and then plated on an agar medium containing 10% sucrose, and colonies were isolated. Whether the finally isolated colonies were resistant to kanamycin was checked, and then whether the argO gene was introduced into the strains having no antibiotic resistance was checked by sequencing (see Schafer et al., Gene 145 (1994) 69-73). As a result, *Corynebacterium glutamicum* mutant strain DS1 having the argO gene introduced thereinto and capable of producing L-arginine was constructed.

### Experimental Example 1. Evaluation of L-arginine productivity of strain into which argO gene has been introduced

The L-arginine productivity of *Corynebacterium glutamicum DA1* constructed in Example 1 and having the argO gene introduced thereinto was compared with that of the parent strain *Corynebacterium glutamicum* 14GR.

Each strain was patched on a flask solid seed medium and cultured at 30°C for 24 hours. Each cultured colony was inoculated into a 10-ml flask titer medium and cultured at 200 rpm at 32°C for 30 hours. The compositions of the media used here are shown in Table 2 below. After completion of the culturing, each culture was diluted 50-fold with distilled water and filtered through a 0.45-µm filter, and then the amount of L-arginine produced was analyzed using high-performance liquid chromatography (HPLC) (Agilent Technologies 1260 Infinity, Agilent Technologies) equipped with a column (DionexIonPac^{™} CS12A) and a UV detector (195 mm), and the results are shown in Table 3 below.

**[Table 2]**

| | |
|---|---|
| Flask solid seed medium (per L) | 10.5 g of 98% glucose, 1 g of beef extract, 4 g of yeast extract, 2 g of polypeptone, 2 g of NaCl, 40 g of (NH₄)₂SO₄ and 20 g of agar |
| Flask titer medium (per L) | 120 g of 98% glucose, 1 g of MgSO₄, 2 g of KH₂PO₄, 45 g of (NH₄)₂SO₄, 20 mg of FeSO₄, 20 mg of MnSO₄, 100 µg of biotin, 100 µg of thiamine, 4 g of YSP and 2 g of urea |

**[Table 3]**

| Strain | OD₆₁₀ | L-arginine (%) | Residual sugar (%) | L-citrulline (%) | L-lysine (%) |
|---|---|---|---|---|---|
| 14GR | 32.0 | 2.37 | 1.43 | 0.026 | 0.023 |
| DA1 | 40.0 | 2.62 | 0.75 | 0.012 | 0.008 |

As shown in Table 3 above, it was confirmed that Corynebacterium glutamicum DA1, into which the argO gene has been introduced, showed an increase of about 10% in L-arginine production due to enhanced arginine export compared to the parent strain *Corynebacterium glutamicum* 14GR, and showed excellent bacterial growth and sugar consumption and reduced production of by-products such as L-citrulline and L-lysine.

So far, the present invention has been described with reference to the preferred embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

### [Accession number]

Depository authority: Korean Culture Center of Microorganisms (KCCM)
Accession number: KCCM13219P
Deposit date: June 29, 2022

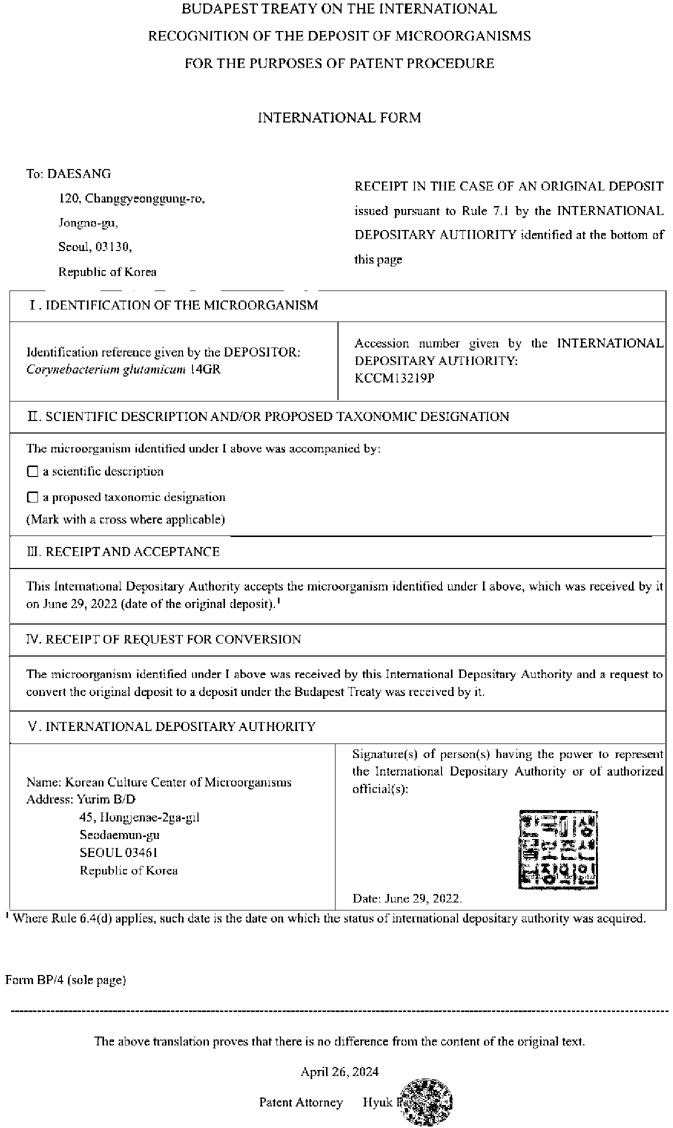
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
120, Changgyeonggung-ro,
Jongno-gu,
Seoul, 03130,
Republic of Korea

RECEIPT IN THE CASE OF AN ORIGINAL DEPOSIT
issued pursuant to Rule 7.1 by the INTERNATIONAL
DEPOSITARY AUTHORITY identified at the bottom of
this page

I . IDENTIFICATION OF THE MICROORGANISM
Identification reference given by the DEPOSITOR:
Corynebacterium glutamicum 14GR | Accession number given by the INTERNATIONAL
DEPOSITARY AUTHORITY:
KCCM13219P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above, which was received by it
on June 29, 2022 (date of the original deposit).¹

IV. RECEIPT OF REQUEST FOR CONVERSION
The microorganism identified under I above was received by this International Depositary Authority and a request to
convert the original deposit to a deposit under the Budapest Treaty was received by it.

V. INTERNATIONAL DEPOSITARY AUTHORITY
Name: Korean Culture Center of Microorganisms
Address: Yurim B/D
45, Hongjenae-2ga-gil
Seodaemun-gu
SEOUL 03461
Republic of Korea | Signature(s) of person(s) having the power to represent
the International Depositary Authority or of authorized
official(s):
Date: June 29, 2022.

¹ Where Rule 6.4(d) applies, such date is the date on which the status of international depositary authority was acquired.

Form BP/4 (sole page)

The above translation proves that there is no difference from the content of the original text.

April 26, 2024

Patent Attorney Hyuk P[?][?][?]

## Claims

1. *A Corynebacterium* sp. microorganism having improved L-arginine productivity by having enhanced activity of an arginine exporter.

2. The *Corynebacterium* sp. microorganism of claim 1, wherein enhancement of the activity of the arginine exporter is achieved by introduction of a gene encoding the arginine exporter.

3. The *Corynebacterium* sp. microorganism of claim 1, wherein the arginine exporter is derived from an *Escherichia* sp. microorganism.

4. The *Corynebacterium* sp. microorganism of claim 1, wherein the arginine exporter comprises the amino acid sequence of SEQ ID NO: 1.

5. The *Corynebacterium* sp. microorganism of claim 1, which is *Corynebacterium glutamicum.*

6. A method for producing L-arginine comprising steps of:
culturing the *Corynebacterium* sp. microorganism of claim 1 in a medium; and
recovering L-arginine from the microorganism or the medium in which the microorganism has been cultured
